(19) Europäisches Patentamt
European Patent Office
Office européen des brevets

(11) Veröffentlichungsnummer : **0 365 777 B1**

# (12) EUROPÄISCHE PATENTSCHRIFT

(45) Veröffentlichungstag der Patentschrift : **17.06.92 Patentblatt 92/25**

(51) Int. Cl.$^5$ : **C07C 69/54,** C07C 67/54, C07C 67/56, C07C 67/03

(21) Anmeldenummer : **89115066.6**

(22) Anmeldetag : **16.08.89**

## (54) Verfahren zur Herstellung von hochsiedenden Acrylaten bzw. Methacrylaten.

(30) Priorität : **22.10.88 DE 3836093**

(43) Veröffentlichungstag der Anmeldung : **02.05.90 Patentblatt 90/18**

(45) Bekanntmachung des Hinweises auf die Patenterteilung : **17.06.92 Patentblatt 92/25**

(84) Benannte Vertragsstaaten : **AT BE CH DE ES FR GB IT LI NL SE**

(56) Entgegenhaltungen :
**DE-A- 1 568 376**
**DE-A- 2 027 443**
**GB-A- 1 174 148**

(73) Patentinhaber : **DEGUSSA AKTIENGESELLSCHAFT**
**Weissfrauenstrasse 9**
**W-6000 Frankfurt 11 (DE)**

(72) Erfinder : **Breuer, Siegfried**
**Johanna-Melber-Weg 27**
**W-6000 Frankfurt/Main 70 (DE)**
Erfinder : **Delle, Heinz, Dr.**
**Mondorfer Weg 32**
**W-6380 Bad Homburg v. d. H. (DE)**
Erfinder : **Rudolph, Udo**
**Grünaustrasse 11**
**W-6450 Hanau 9 (DE)**

EP 0 365 777 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von hochsiedenden Acrylaten bzw. Methacrylaten mit niedrigen Farbzahlen und hoher Reinheit. Die Produkte werden durch Umesterung von (Meth)-Acrylsäure-Methylester mit entsprechenden Alkoholen dargestellt unter Einsatz von Alkali-, Erdalkali, Titan- oder Zirkon-Alkoholaten als Katalysatoren und Polymerisationsinhibitoren sowie Schleppmitteln für das Austreiben des bei der Reaktion freiwerdenden Methanols.

Verfahren dieser Art sind in verschiedenen Varianten bekannt.

Diese zielen jedoch im allgemeinen nur auf einen möglichst hohen Umsatz ab (DE-OS 2805 702) und lassen bei den höhersiedenden, z. B. Trimethylolpropantrimethacrylat, eine destillative Reinigung außer Betracht (DE-OS 2744 641).

Apha-Farbzahlen nach Hazen von 15 (DIN 55409 ) kann man bei der Herstellung von höhermolekularen Alkylacrylaten bzw. -methacrylaten erzielen, wenn man gemäß DE-OS 2317 226 die Umesterung in Gegenwart einer kleinen Menge absorptionsfähiger Kohle als Polymerisationsinhibitor durchführt.

Folgt man dieser Patentschrift sind die erwünschten Reinheitgrade mit Hilfe einer Vakuumdestillation, wie sie in der GB-PS 962 928 vorgeschlagen wird, nicht zu erreichen.

Aufgabe der Erfindung ist ein Verfahren zur Herstellung von hochsiedenden Acrylaten bzw. Methacrylaten mit noch niedrigeren Farbzahlen und besonders hoher Reinheit.

Gegenstand der Erfindung ist ein Verfahren zur Herstellung von hochsiedenden Acrylaten bzw. Methacrylaten mit niedrigen Farbzahlen durch Umesterung von Methylacrylat bzw. Methacrylat mit mehrwertigen Alkoholen auf an sich bekannten Wegen, das dadurch gekennzeichnet ist, daß man nach der Abtrennung des jeweils eingesetzten Katalysators das Produkt bei einer Sumpftemperatur von 110 bis 170°C und einem Druck von 0,01 bis 10 mbar einer Kurzwegverdampfung unterwirft.

Unter dem Begriff hochsiedend sind Verbindungen zusammengefaßt, deren Siedepunkt unter Normaldruck 160 C, insbesondere 190 °C übersteigt und 400 °C erreichen kann. Erfindungsgemäß herstellbare höhermolekulare Ester mit einem Molekulargewicht bis ca. 400 entsprechen der allgemeinen Formel (I)

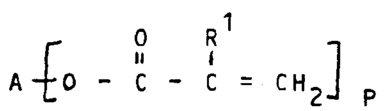

(I),

in der bedeuten:
$R^1$: Wasserstoff oder Methyl,
mit $_P$= 3
A: ein geradkettiger oder verzweigter, gegebenenfalls 1 oder 2 Sauerstoffbrücken enthaltender Rest mit 4 bis 6 Kohlenstoffatomen,
mit $_P$= 2
A: ein geradkettiger oder verzweigter, 0 oder 2 bis 3 Sauerstoffbrücken enthaltender Rest mit 2 bis 8 Kohlenstoffatomen.

Bevorzugt sind Ester der Alkohole 1,4 Butandiol, Tripropylenglykol, Triethylenglykol, sym. Dimethylethylenglykol, Tetraethylenglykol, 2,2,4-Trimethyl-1,3-propandiol, Trimethylolethan und Trimethylolpropan.

Verfahren zur Umesterung sind bekannt, wie in der Einleitung schon gezeigt wurde. Man geht im allgemeinen so vor, daß man die Methylester mit den Alkoholen bei Temperaturen von 60 bis 140 °C in Gegenwart von geeigneten Umesterungskatalysatoren und Polymerisationsinhibitoren umsetzt.

Als letztere haben sich im vorliegenden Verfahren insbesondere Phenothiazin und/oder dessen Derivate, bevorzugt Methylenblau bewährt.

Diese werden in einer Konzentration von 50 bis 2000 ppm, bezogen auf das Reaktionsgemisch Alkohol/(Meth-)Acrylat eingesetzt.

Der während der Reaktion entstehende Methylalkohol wird als azeotropes Gemisch mit einem Schleppmittel, wie z. B. n-Hexan oder Cyclohexan kontinuierlich abgezogen und vorzugsweise in einen über einem Phasentrenngefäß geschlossenen Waschwasserkreislauf eingespeist.

Dabei tritt das Methanol praktisch quantitativ in die Wasserphase über, während die sich bildende Hexanphase im Wärmeverbund mit dem Kopfkondensat wieder auf den Kolonnenkopf geführt wird.

Die Aufarbietung der Waschwasserphase erfolgt nach bekanntem Muster.

Die Umesterungsreaktion wird, bevorzugt unter Durchleiten von sauerstoffhaltigen Gasen, vorzugsweise Luft, durchgeführt.

Nach Beendigung der Umsetzung trennt man den Umesterungskatalysator in geeigneter Weise ab. Alkoholate wie z. B. Alkyltitanate werden hydrolisiert und das ausfallende Hydroxid gegebenenfalls unter Zusatz von Filterhilfsmitteln abfiltriert.

Bei der Verseifung entstehendes Wasser wird als Azeotrop ausgeschleust.

Verwendet man Lithiumsalze als Umesterungskatalysatoren sind entsprechende Maßnahmen zu ergreifen (DE-OS 2744 641).

Anschließend erfolgt die destillative Reinigung, in deren erstem Schritt z. B. über einen Fallfilmverdampfer überschüssige Ausgangsprodukte und das Schleppmittel abgetrennt werden.

Das so erhaltene Rohprodukt wird in dem zweiten Schritt der Kurzwegverdampfung bzw. -destillation unterworfen.

Sollten im Reindestillat noch mitgeschleppte Spuren z. B. des Stabilisators Methylenblau enthalten sein, können diese ohne Schwierigkeiten beim Durchlauf durch einen Aktivkohlefilter entfernt werden.

Man erhält so Produkte mit einer Reinheit von > 98 % (gaschromatographische Analyse) und einer Apha-Zahl von 0 bis 5.

Es ist natürlich auch möglich, niedrigersiedende Ester auf diesem Wege zu reinigen.

Beispiel 1

600 g Triethylenglycol (TEG), 1200 g Methylmethacrylat (MMA), 0,14 g Hydrochinon-monomethylether (MEHQ), 1,0 g Methylenblau und 300 g n-Hexan werden in einen 3 l Doppelmantel-Glasreaktor (Heiz/Kühlmantel) mit aufgesetzter 10-Boden-Kolonne eingebracht. DAs Gemisch wird auf Siedetemperatur aufgeheizt, um zunächst im Gemisch enthaltene Wasserspuren azeotrop mit n-Hexan auszuschleusen. Das Kopfkondensat läuft in einen über einem Phasentrenngefäß geschlossenen Waschwasserkreislauf, in dem das ausgetragene Wasser verbleibt, während das n-Hexan aus dem Trenngefäß überläuft und auf den Kopf der Kolonne zurückgeführt wird. In das Reaktionsgemisch sind ca. 5 l/h Luftsauerstoff zur Stabilisierung einzuspeisen. Nach der Entwässerungsphase werden dem Reaktionsgemisch 25 g Alkyltitanat zugesetzt, wodurch die Umesterungsreaktion gestartet wird. Das freigesetzt Methanol bildet mit dem n-Hexan ein bei ca. 50 °C siedendes Azeotrop, so daß Methanol schnell in den Kolonnenkopf geschleppt wird, dessen Temperatur ca. 68 °C beträgt. Das Kopfkondensat (Hexan + Methanol) wird vollständig in den bereits erwähnten Waschkreislauf eingespeist. Dabei tritt das Methanol praktisch quantitativ in die Wasserphase über, während die gebildete Hexanphase im Wärmeverbund mit dem Kopfkondensat wieder auf den Kolonnenkopf geführt wird. Die Waschwasserphase reichert sich im Verlauf der Umesterungsreaktion mit Methanol an und sollte ab ca. 50 % Methanol kontinuierlich ausgeschleust werden bei gleichzeitiger Einspeisung von Frischwasser in den Kreislauf. Der vollständige Umsatz des TEG muß mit Hilfe gaschromatografischer Analytik festgestellt werden. Bei Reaktionsende (Reaktionstemperatur etwa 120 °C) wird auf ca. 80 °C abgekühlt. Es erfolgt die Zugabe von 50 g 10 %iger $H_2SO_4$ zur Hydrolyse des Alkyltitanats, die etwa 30 Minuten dauert. Danach werden 20 g $CaCO_3$ zugegeben. Es bilden sich Titanhydroxid und $CaSO_4$. Freies Restwasser wird nach nochmaligem Aufheizen azeotrop - wie bei der bereits beschriebenen Entwässerung - ausgeschleust. Der im Reaktionsgemisch befindliche Feststoffanteil kann - gegebenenfalls nach Zusatz von Filterhilfsmitteln - mit üblichen Filterapparaten quantitativ separiert werden. Das Filtrat wird der destillativen Aufarbeitung zugeführt. Die Destillation erfolgt in zwei Stufen. Zunächst werden in der ersten Stufe über einen Fallfilmverdampfer Überschuß-MMA undHexan bis auf einen Restgehalt von < 0,2 % im Rohprodukt (Sumpf) abgetrennt. Die Destillation erfolgt bei ca. 120 °C Ablauftemperatur des Rohproduktes und ca. 30 mbar Betriebsdruck. Das Destillat (MMA und Hexan) wird in den nächsten Umesterungsbatch rezykliert. Das Rohprodukt wird schließlich in der zweiten Stufe bei ca. 0,1 mbar und ca. 150

°C (Sumpfablauftemperatur) einer Kurzwegdestillation unterzogen. Der Sumpf, ca. 5% der Destillatmenge, geht in den nächsten Umesterungsbatch zurück. Das Reindestillat läuft nach Abkühlung zur Adsorption gegebenenfalls mitgeschleppter Spuren Methylenblaus durch ein Aktivkohlefilter in den Produkttank. Das Produkt besitzt eine Reinheit von > 98 % (GC). Die Aphazahl beträgt 0 ./. 5.

## Patentansprüche

1. Verfahren zur Herstellung von hochsiedenden Acrylaten bzw. Methacrylaten mit niedrigen Farbzahlen durch Umesterung von Methylacrylat bzw. Methacrylat mit mehrwertigen Alkoholen auf an sich bekannten Wegen, dadurch gekennzeichnet, daß man nach der Abtrennung des jeweils eingesetzten Katalysators das Produkt bei einer Sumpftemperatur von 110 bis 170 °C und einem Druck von 0,01 bis10 mbar einer Kurzwegverdampfung unterwirft.

2. Verfahren gemäß Anspruch 1, dadurch gekennzeichnet, daß man das Reindestillat über einem Aktivkohlefilter reinigt.

## Claims

1. A process for the preparation of high boiling acrylates or methacrylates having low colour values by the trans-esterification of methyl acrylate or methacrylate with polyhydric alcohols by methods known per se, characterised in that after removal of the catalyst used, the product is subjected to molecular evaporation at a sump temperature of from 110 to 170°C and a pressure of from 0.01 to 10 mbar.

2. A process according to Claim 1, characterised in that the pure distillate is purified through an active charcoal filter.

## Revendications

1. Procédé de production d'acrylate ou méthacrylate à point d'ébullition élevé avec des indices de couleur bas par transestérification de méthylacrylate ou méthacrylate avec des alcools à plusieurs valences par des méthodes connues en soi, procédé caractérisé en ce qu'après la séparation du catalyseur respectivement utilisé, on soumet le produit à une évaporation flash à une température de réservoir de 110°C à 170°C et une pression de 0,01 à 10 mbars.

2. Procédé selon la revendication 1, caractérisé en ce qu'on affine le distillat pur sur un filtre de charbon actif.